# NEW EUROPEAN PATENT SPECIFICATION

(11) **EP 1 261 259 B2**
(45) Date of publication and mention of the opposition decision: **06.06.2012**
(45) Mention of the grant of the patent: 09.11.2005
(21) Application number: 01910035.3
(22) Date of filing: 09.03.2001
(51) Int. Cl.: A23K 1/14, A23K 1/18, A23K 1/16

(54) **TREATMENT OF INFECTION IN ANIMALS**
BEHANDLUNG VON INFEKTIONEN IN TIEREN
TRAITEMENT D'INFECTIONS CHEZ DES ANIMAUX

(30) Priority: 10.03.2000 WO PCT/GB00/00890; 22.05.2000 GB 0012401; 11.09.2000 GB 0022210
(43) Date of publication of application: 04.12.2002
(73) Proprietor: MARS UK LIMITED, Slough, Berkshire SL1 4LG (GB)
(72) Inventor: BAILLON, M-L, c/o Waltham Cent for Pet Nutritition, Leicestershire LE14 4RT (GB); GIFFARD, Catriona Julie, Waltham-on-the-Wolds, Leicester LE14 (GB)
(74) Representative: Cornish, Kristina Victoria Joy
(86) International application number: PCT/GB2001/001036
(87) International publication number: WO 2001/065949

(56) References cited:
- WO-A-91/18521
- WO-A-96/39046
- JP- - 08 713 055
- PATENT ABSTRACTS OF JAPAN vol. 1996, no. 11, 29 November 1996 (1996-11-29) & JP 08 173055 A (HOSHIDA SHINICHI;SEIBUTSU KAGAKU SANGYO KENKYUSHO:KK; YOUNICHI KAGAKU), 9 July 1996 (1996-07-09)
- M DIEZ ET AL: "Etude des fibres alimentaires chez le chien: présentation des résultats de 7 essais expérimentaux" ANNALES DES MEDECINE VETERINAIRE,FR,XX, vol. 142, no. 3, 1 January 1998 (1998-01-01), pages 185-198,200-201, XP002093241
- E.A.FLICKINGER, ET AL J.NUTR.130: February 2000, pages 1267 - 1273
- G.R.GIBSON, ET AL J.NUTR.130 2000, pages 3915 - 3955
- A.H.SPARKES ET AL AJUR 1998, pages 436 - 440
- M.D.WILLARD ET AL AM.J.VET.R.S 1994,
- A.H.SPARKES ET AL AJVR, 59(4) 1998, pages 431 - 435

## Description

The present invention relates to the use of coconut endosperm fibre in the manufacture of a composition for treating or preventing pathogenic bacteria in the large intestine of a dog or a cat.

Presence of pathogenic bacteria (including infection) of the large intestine in a pet animal is concerning. Particular pathogenic bacteria which infect the large intestine include *Campylobacter* and pathogenic *Escherichia coli.* The bacterial species responsible for the majority of human bacterial gastrointestinal infections is *Campylobacter jejuni.* This species is also the main cause of concern for cats and dogs. The species can act as a pathogen in young dogs and cats and is likely to be opportunistic in older animals. Clinical illness in dogs manifests itself as diarrhoea ranging from mild to mucus laden bloody diarrhoea, tenesmus, vomiting, anorexia and depression.

A major concern regarding *Campylobacter* infection in pet animals is the zoonotic risk which carriage and excretion of the organism represents. It has been estimated that 5% of all human *Campylobacter jejuni* induced diarrhoea results from exposure to infected cats or dogs. A number of more recent studies quote dog ownership as a significant risk factor for becoming ill with *Campylobacter.* A study carried out in Christchurch, New Zealand found that household contact with dogs carried a risk of 1.25 to 2 times normal for contracting *Campylobacter.*

*E. coli* is a Gram negative facultative anaerobic bacillus. In general it leads a synbiotic life with its host causing it no harm. However specific groups are known to cause gastroinestinal disease and are classified into categories as defined by their virulence mechanisms. Enteropathogenic and verocytotoxigenic strains of *E. coli* are particularly important in causing acute and chronic diarrhoea in dogs. The verocytotoxigenic *E. coli* are thought to be important in diarrhoeic as well as healthy cats and these animals are likely to act as a reservoir of infection for humans.

*Salmonella* organisms are Gram negative, facultative anaerobic bacteria that are able to survive intracellularly. *Salmonella* can cause clinical and subclinical infections in dogs and cats as well as humans. This makes them a key organism of interest.

Various studies have found *Salmonella* to be carried by between 1 and 30% of healthy domestic pet dogs and between 1 and 18% of healthy domestic pet cats. This data is dependent on the survey and whether *Salmonella* could be cultured from the faeces of animals both with and without diarrhoea.

Clinical infections of *Salmonella* in animals often display signs of mild to severe gastroenteritis. In dogs, symptoms most often reported are diarrhoea, vomiting, fever, malaise, anorexia, vaginal discharge and sometimes abortion. In cats, diarrhoea, vomiting, fever, malaise and anorexia are the predominant symptoms reported. Recovery from acute Salmonellosis typically occurs within 1 week but can take up to 3 to 4 weeks. Shedding of *Salmonella* in faeces can continue for 3 to 6 weeks and can be a source of infection for human family members.

Due to the long term shedding of *Salmonella,* animals are important vectors for non-foodborne infections in humans. Dogs have a greater zoonotic potential than cats although cats have been shown to shed organisms orally, conjunctivally and faecally. Contact with faeces from infected pets is an important source of infection to young children.

The location of *Salmonella* infection is in the small intestine but due to the potential zoonotic risk from long term shedding of *Salmonella* in faeces from dogs, a model of the large intestine has been used for this investigation. If viable *Salmonella* numbers can be reduced while in the large intestine then the duration of shedding can be reduced. Reducing the time that *Salmonella* is shed in dog faeces also reduces the chance of human contact with the pathogen.

JP 08173055 discloses the use of mannose polysaccharide to prevent salmonella bacterial contamination in livestock animals.

Accordingly, there is a need for a means of preventing or treating pathogenic bacteria in the large intestine of pet animals to eliminate the aforementioned risks. Current treatments for *Campylobacter* and pathogenic *E. coli* infections and the presence of *Salmonella* involve administering antibiotics to the pet animals. There are concerns that the continued use of antibiotics in treating *Campylobacter* infections may lead to the emergence of antibiotic resistant strains of this organism and may have an effect on the long-term health of pet animals (which would reduce or eliminate treatment options). A need therefore exists to find an alternative to antibiotic treatment for infected or infectable animals. The present invention provides such a means.

According to the present invention there is provided the use of coconut endosperm fibre in the manufacture of a composition for the prevention or treatment of pathogenic bacteria in the large intestine of a dog or a cat.

In general, non-digestible carbohydrates comprise those compounds which are not digested by mammals but may be metabolised by intestinal bacterial species belonging to the normal microflora for example, bifidobacteria and lactobacilli.

Oligosaccharides are naturally occurring compounds which can be found in a variety of fruits and vegetables such as bananas, tomatoes, artichokes, onions, garlic and cereals (eg wheat and barley).

Fresh coconut endosperm has a typical nutrient distribution of water (35 %), oil (44 %), protein (6%), sugars (7%), fibre (3%) and ash (1%). However, the form of the coconut endosperm fibre for use according to all aspects of the present invention is not limiting. The coconut endosperm fibre may be fresh or in any other form such as copra, defatted copra (also referred to, amongst others, as copra cake, copra presscake or copra meal) coconut flour, defatted coconut flour, full or defatted desiccated coconut, copra.

Copra is a particularly suitable source of coconut endosperm fibre for use according to the present invention. Copra is dried coconut endosperm (usually sundried). Defatted copra is also particularly suitable. Defatted copra is the typical result of coconut endosperm which has been dried and had the coconut oil mechanically removed. Defatted copra cake is obtained by first obtaining copra, then crushing the copra through a press or expeller to remove most of the oil. The residue remaining is termed copra cake, copra presscake, or copra meal.

One of the products of complex carbohydrate fermentation in the large intestine by normal microflora are short chain fatty acids (SCFAs). The production of SCFAs by the gut microflora results in a decrease in the pH of the lumen of the large intestine/colon. However, not all intestinal bacteria can metabolise non-digestible carbohydrates. Studies show that pathogenic bacteria are unable to process non-digestible carbohydrates. For this reason these non-digestible carbohydrates in the gut are involved in selectively stimulating the growth of the gut microflora creating a healthier gastrointestinal environment.

The coconut endosperm fibre of the invention selectively stimulates the growth and/or activity of one or a limited number of beneficial bacteria in the large intestine of a pet animal. The term "beneficial bacteria" refers to those species of bacteria which have a beneficial effect on the host organism.

The beneficial species of bacteria typically comprise one of more of bifidobacteria and lactobacilli.

The coconut endosperm fibre of the present invention may be a "prebiotic". A prebiotic is defined in the art as a non-digestible food ingredient that beneficially affects the host by selectively stimulating the growth and/or activity of one or a limited number of bacteria in the colon, that have the potential to improve health.

Embodiments of the present invention have been shown to have potential in lowering the pH of the large intestine. Using an oligosaccharide, a lowering of the pH in the large intestine model from 7.25 to 5.5 was observed. In the large intestine, a lowering of the pH affects the survival of pathogenic bacteria. A lowering of pH from 7.25 to 5.5 as a result of the inclusion of FOS and/or GOS, is sufficient to eliminate viable *Campylobacter jejuni* bacteria. In contrast, the inclusion of coconut endosperm fibre in the large intestine results in the elimination of viable *Campylobacter jejuni* bacteria without a significant change in the pH of the large intestine (the pH was lowered from 7.0 to 6.5 in the large intestine model).

The present invention provides feeding dogs or cats with a composition comprising a coconut endosperm fibre to reduce or eliminate viable pathogenic bacteria in the large intestine.

The pathogenic bacteria of the present invention typically include one of more of *Campylobacter,* pathogenic *Clostridium, Salmonella* and pathogenic *Escherichia coli,* such as verocytotoxigenic *E.coli*, for example *E. coli* 0157. Of particular interest for the present invention are the species *Campylobacter jejuni* and *Salmonella enterica* (including *S. enterica* serotype Typhimurium). *Campylobacter jejuni* may cause clinical illnesses including diarrhoea, tenesmus, vomiting, anorexia, depression, inflammatory intestinal disease and other intestinal disorders.

The pet animal is a cat or a dog. Cats and dogs according to the present invention are preferably *Felis domesticus or Canis domesticus.*

The composition for the prevention or treatment of a pathogenic bacteria in the large intestine of the pet animal is preferably a pet food product The form or type of the pet food product is not limiting. It may be packaged. In this way, the consumer is able to identify, from the packaging, the ingredients in the food product and confirm that it is suitable for the particular pet animal in question. The packaging may be metal (usually in the form of a tin or flexifoil), plastic, paper or card. The pet food product may be a dry, semi-moist or a moist (wet) product. Wet food includes food which is sold in tins and has a moisture content of 70 to 90%. Dry food includes food having a similar composition, but with 5 to 15% moisture and presented as small biscuit-like kibbles. Semi-moist products have a moisture content between wet and dry products. The moisture content is in the range of from 15 to 70%. The amount of moisture in any product may influence the type of packaging which can be used or is required.

In combination with the coconut endosperm fibre, the remaining components of the pet food product are not essential to the invention and typical standard products can be combined with the required coconut endosperm fibre. Most preferably, the combined ingredients of the pet food product according to the invention provide all of the recommended vitamins and minerals for the particular pet in question, (a complete and balanced food), for example, as described in National Research Council, 1985, Nutritional Requirements for Dogs, National Academy Press, Washington D.C. (ISBN: 0-309-03496-5); National Research Council, 1986, Nutritional Requirements of Cats, National Academy Press, Washington D.C. (ISBN: 0-309-03682-8) or Association of American Feed Control Officials, Official Publication 1996.

The pet food product encompasses any product which a pet consumes in its diet. Thus, the invention covers standard food products as well as pet food snacks (for example, snack bars, pet chew, crunchy treat, cereal bars, snacks, biscuits and sweet products). The food product is preferably a cooked product. It may be in the form of a gelatinized starch matrix. It may be in the form of chunks in gravy, jelly, loaf or water. It may incorporate meat or animal derived material (such as beef, chicken, turkey, lamb, pork, fish, blood plasma, marrow bone etc or one or more thereof). The product alternatively may be meat free (preferably including a meat substitute such as soya, maize gluten or a soya product) in order to provide a protein source. The product may contain additional protein sources such as soya protein concentrate, milk proteins, gluten etc. The product may also contain a starch source such as one or more grains (e.g. wheat, corn, rice, oats, barley etc), or may be starch free. A typical dry dog or cat food contains about 20-30% crude protein and about 10-20% fat, the remainder being carbohydrate, including dietary fibre and ash. A typical wet, semi-wet or moist product contains (on a dry matter basis) about 40% fat (from 20 to 50%), 50% protein (from 40 to 60%) and the remainder being fibre and ash.

The pet food product is preferably a commercial pet food product. Such a product is preferably sold as a product for feeding to a pet animal, in particular a pet cat or a pet dog.

The pet food product may comprise from 0.1 to 5 weight% of an oligosaccharide, most preferably from 0.1 to 2 weight% (on a dry matter basis).

The level of non-digestible fibre incorporated into a pet food product such as coconut endosperm fibre is not limiting. Preferably, the fibre component is present in the pet food product at a level of from approximately 0.15 to 8% on a dry matter basis, preferably 0.15 to 5% on a dry matter basis as measured by the Englyst method (as defined in Englyst H.N., and Cumming J.H. (1984), simplified method for the measurement of total non-starch polysaccharides by gas-liquid chromatography of constituent sugars as alditol acetates. *Analyst.* 109, 937-942, and incorporated herein by reference). The levels, as calculated by this method, may go from 0.15% up to 5%, 6%, 7% or 8%. The lower limit may be from 1.5%, 2% or 3%. A description of the Englyst method is described in Appendix 1. In principle, starch is removed enzymatically after solubilisation and NSP is measured as the sum of the constituent sugars released by acid hydrolysis. The starch component of the fibre source is gelatinised by boiling in hot water and is then removed with alph-amylase and pullulanase. Starch and modified, or resistant starch are dispersed in DMSO. Three samples are then subjected to complementary procedures measuring (I) total NSP (ii) water-soluble NSP and (iii) cellulose. Components are hydrolysed in each case with sulphuric acid. The constituent sugars are converted to alditols and are measured as their alditol acetates using gas-liquid chromatography (GLC). Values for total dietary fibre as well as insoluble and soluble fractions can be obtained. Cellulose can be measured separately and the non-cellulose polysaccharides are characterised by measurement of the individual monosaccharides.

The incorporation of the level of coconut endosperm fibre according to the invention (which may differ according to the form of the coconut endosperm, for example copra or desiccated coconut) can easily be determined by identifying the amount of dietary fibre in the particular form of the coconut endosperm fibre. For example, according to the Englyst method (*Supra*) defatted copra contains approximately 33.5% total dietary fibre. Accordingly, the preferred amount of defatted copra in a pet food product in order to provide a preferred fibre level of from approximately 0.15 to 5% on a dry matter basis according to the first aspect of the invention is at a level from approximately 0.5 to 15% on a dry matter basis of the pet food product.

Without limiting the present invention, the addition of coconut endosperm fibre into a pet food product is believed to maintain good health of the large intestine or improve it, generally achieved by optimising the conditions for growth and multiplication of non-harmful bacteria in the large intestine and/or by lowering the pH of the lumen of the large intestine thereby reducing the amount of harmful bacteria in the large intestine.

The composition is preferably administered to a pet animal in need of the prevention or treatment of a pathogenic bacteria in its large intestine. This may be to, for example a young pet animal, such as a puppy, or an older pet animal. Where the composition is a pet food product, the pet food product may be administered in a dietary regime in accordance with the usual dietary regime of the pet animal. The pet food product may comprise 100% of the diet of the pet animal or a lesser proportion, depending on the level of prevention or treatment required. The pet food product allows the coconut endosperm fibre to be administered with ease thus avoiding a need to supplement the pet animal's food. In addition the pet food product can be administered by the animal's owner thus avoiding constant veterinary supervision. The pet food product may be available at any outlet selling pet food products or may be available from a veterinarian. The pet food product may be as described above according to the first aspect of the invention.

As used herein, the term "administration" also includes feeding or any other method of oral administration. Other means of administration may include tablets, capsules, injection, suppositories or any other suitable means.

The invention will now be described with reference to the following non-limiting figure, in which:

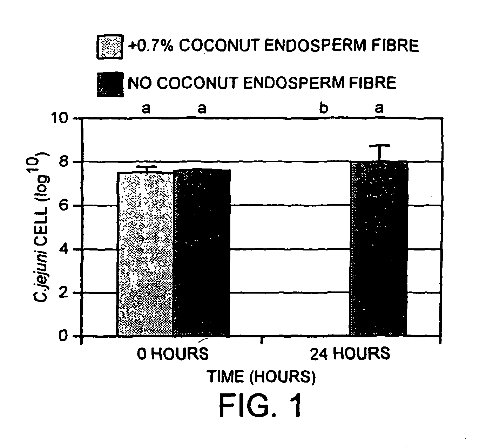

Figure 1 is a graph of *C. jejuni* cells at 0 and at 24 hours in incubation with or without coconut endosperm fibre.

The invention will now be described with reference to the following, non-limiting example:

### EXAMPLE

### The effect of coconut endosperm fibre on the survival of Campylobacter jejuni in a model of the canine intestine

### METHOD

Investigation into the effect of coconut endosperm fibre on the survival of *Campylobacter* in the canine intestine.

### Summary

- *Campylobacter* is one of the most predominant gastrointestinal pathogens causing both clinical and non-clinical infections in dogs.
- An *in vitro* model of the canine large intestine has been developed to test the effect of novel fibres on the survival of canine bacterial pathogens.
- Inclusion of coconut endosperm fibre in this model resulted in the elimination of viable *Campylobacter jejuni* cells from the system.

### Methods

*1. Campylobacter jejuni* cells were grown from stock cultures and cultured at 37°C under microaerobic conditions (5% O₂, 10% CO₂ and 85% N₂). Liquid cultures were grown in 20ml volumes in 50ml conical flasks shaken on an orbital shaker. Overnight cultures grown in Mueller Hinton (MH) broth (Oxoid) were adjusted to A₆₀₀ 1.0 before inclusion in the assay.
2. Flasks were set up with 200ml MH broth, 1ml of the adjusted *Campylobacter jejuni* culture, and 2g fresh faeces. To test flasks, 0.7% (w/v) copra cake was added and swirled to mix. Control flasks had no further additions.
3. Flasks were sampled at the start (0 hours) and end (24 hours) of the experiment to determine viable counts of *Campylobacter jejuni* cells by serially diluting samples from the flasks and plating dilutions on to *Campylobacter* selective agar (LabM). Plates were incubated microaerobically for 48 hours, after which viable numbers were determined.
4. At the end of the experiment the pH of the mixture in each flask was determined using Multistix (Bayer).
5. The experiment was conducted six times using a faecal sample from a different dog each time. All dogs were fed a commercially available premium (complete and balanced) dry food for the duration of the study.

### RESULTS

After a 24 hour microaerobic incubation, no viable *Campylobacter jejuni* cells could be recovered from flasks that had coconut endosperm fibre added. In contrast, *Campylobacter jejuni* cells were recovered from the flasks that contained no coconut endosperm fibre at around 10⁸ cells per ml. The results from the 6 individual experiments are shown in the table below and in the graph in Figure 1.

**Table**

| Numbers of viable *Campylobacter jejuni* cells (log₁₀) recovered from the model of the canine large intestine with and without the addition of coconut endosperm fibre. | | | | |
|---|---|---|---|---|
| **<-----log₁₀ colony forming units of *Campylobacter jejuni*----->** | | | | |
| **Faecal Sample (Dog No)** | **0 hours < +0.7%** | **0 hours Coconut None** | **24 hours Endosperm +0.7%** | **24 hours Fibre > None** |
| 1 | 7.76 | 7.75 | 0 | 7.25 |
| 2 | 7.33 | 7.64 | 0 | 8.56 |
| 3 | 7.77 | 7.75 | 0 | 7.17 |
| 4 | 7.72 | 7.74 | 0 | 7.47 |
| 5 | 7.67 | 7.71 | 0 | 8.59 |
| 6 | 7.16 | 7.38 | 0 | 8.97 |
| **Mean** | **7.57** | **7.66** | **0** | **8** |
| STD | 0.26 | 0.14 | 0 | 0.79 |

Figure 1 shows a graph of the effect of the inclusion of coconut endosperm fibre in the canine large intestine model on the survival of *Campylobacter jejuni*. Letters denote statistically significant difference (p < 0.05).

Recorded pH after 24 hours incubation with coconut endosperm fibre included and omitted from the system for each dog.

| Dog No. | +0.7% Coconut endosperm fibre | No Coconut endosperm fibre |
|---|---|---|
| 1 | 6.75 | 7.5 |
| 2 | 6.25 | 7.5 |
| 3 | 6.75 | 7.5 |
| 4 | 6.25 | 7.5 |
| 5 | 6.25 | 7.5 |
| 6 | 6.25 | 7.5 |

At the end of the incubation period the pH of the solutions in each flask was measured and was found to be 7.5 when coconut endosperm fibre was omitted from the system (SD of 0). When coconut endosperm fibre was included in the model, the pH was found to be 6.42 (SD of 0.26).

### CONCLUSION

Inclusion of coconut endosperm fibre in a model of the canine large intestine resulted in the elimination of viable *Campylobacter jejuni* cells. With no coconut endosperm fibre added to the system, *Campylobacter jejuni* cells showed no loss in viability for the duration of the experiment. As a pH range of 6.5 to 7.5 is optimum for *Campylobacter,* it is unlikely that the difference in pH observed between the two conditions was responsible for the difference observed in survival. Instead, it is likely that the non-pathogenic, saccharolytic bacteria present in the faeces metabolise the coconut endosperm fibre. *Campylobacter jejuni* is incapable of fermenting carbohydrates, thus the coconut endosperm fibre being present gives the non-pathogenic, saccharolytic bacteria an advantage.

### Appendix 1

The Englyst method, from Englyst and Cummings (*Supra*).

### Experimental

### Apparatus

The fractionation procedure was carried out in 50-60ml screw-topped glass centrifuge tubes as previously described. Gas-liquid chromatography was performed with a Pye Unicam Series 204 chromatograph, fitted with a flame-ionisation detector. A 2.1m x 2mm i.d. glass column packed with Supelcoport (100-200 mesh) coated with 3% SP 2330 was used. The column temperature was 215°C (isothermal) and the injector and detector temperatures were 250°C. The carrier gas (nitrogen) flow-rate was 20ml min-¹.

### Reagents

High purity certified reagents were used for all analyses. Enzyme preparations were as follows: hog pancreatic α-amylase, E.C.3.2.1.1. (Sigma, Cat. No. A4268); pullulanase, E.C.3.2.1.41. (Boehringer, Cat. No. 108944).

### Method

The sequence of steps in the procedure is summarised below.

### Pre-treatment of sample

As far as possible, foods should be analysed without any pre-treatment. If there are problems in taking a representative sample, foods with a low water content can be ball milled for 2-3 minutes, and those with a higher water content homogenised, or freeze-dried and ball milled.

### Sample Mass

Accurately weigh between 50 and 1,000mg of sample, containing not more than 150mg of starch and 50mg of NSP, into a 50-60ml screw-top centrifuge tube and add a stirrer.

### Fat Extraction and Drying

Samples with dry matter between 90 and 100 % and with less than 203 % of fat can be analysed directly. Otherwise, add 40ml of acetone, mix for 30 minutes by using a magnetic stirrer, centrifuge and remove by aspiration as much of the supernatant as possible without disturbing the residue. Place the tubes in a water bath at 65°C on a magnetic stirrer hot plate and mix the residue for a few minutes until it appears to be dry. The beaker can be covered and the acetone vapour removed by water pump.

### Dispersion of the Starch

Add 2ml of DMSO, cap the tube and heat it in a boiling water bath for 1 hour, timed from when re-boiling commences, stirring continuously. Then, without cooling, add 8ml of 0.1M sodium acetate buffer pH5.2, at 50°C and vortex mix immediately.

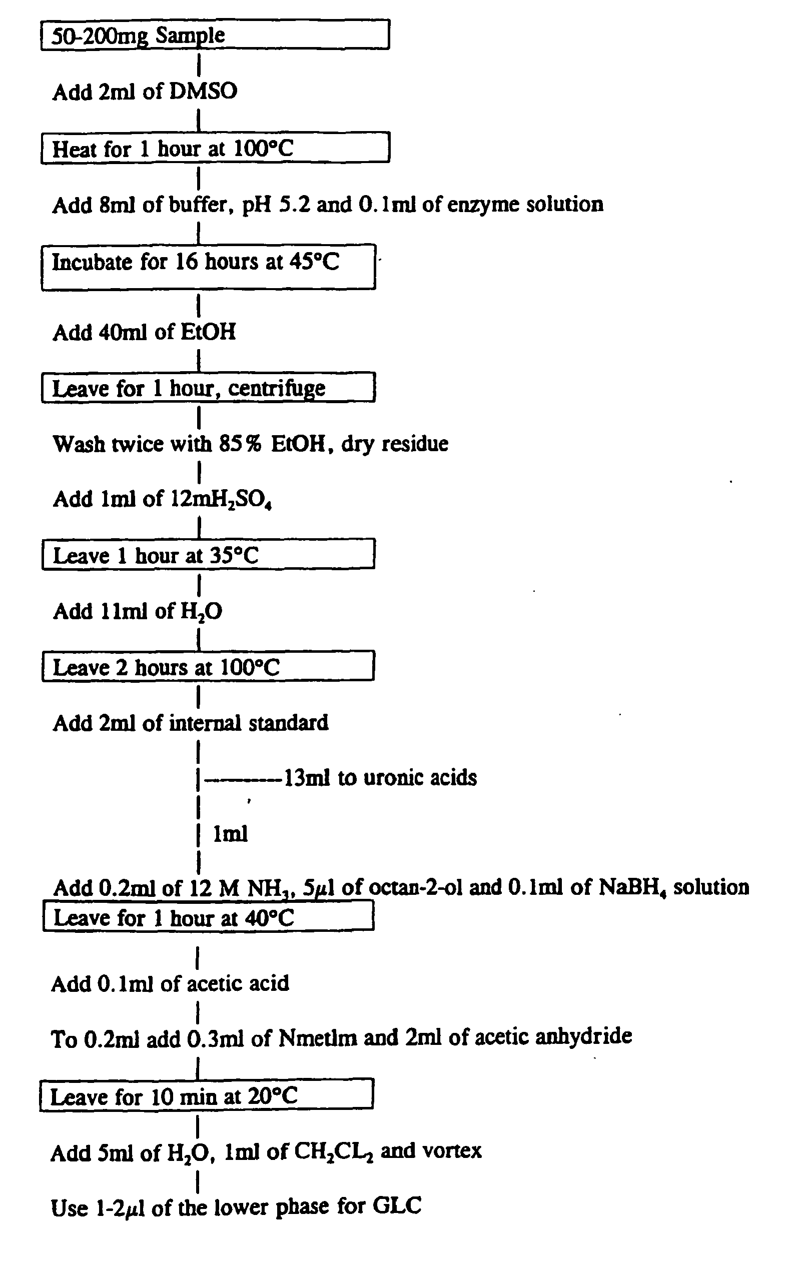

### Procedure for the analysis of non-starch polysaccharides (NSP)

### Enzyme Hydrolysis of the Starch

Cool the tube to 45°C and immediately add 0.1ml of an enzyme solution containing 5,000 units of α-amylase and 5 units of pullulanase per ml of acetate buffer at pH 5.2. Incubate the samples at 45°C for 16-18 hours, preferably mixing continuously as described previously.

Following the enzyme treatment, add 40ml of absolute ethanol, mix well and leave to stand for 1 hour at room temperature. Centrifuge for 10 minutes or until a clear supernatant liquid is obtained. Removed by aspiration as much of the supernatant liquid as possible, without disturbing the residue, and discard it. Wash the residue twice with 50ml of 85 % ethanol by mixing to form a suspension, centrifuging until clear and removing the supernatant liquid as before. Add 40ml of acetone to the washed residue, stir for 5 minutes and then centrifuge. Remove the supernatant liquid by aspiration and dry the residue as described under *Fat extraction and drying.*

### Acid hydrolysis of the residue from enzymic digestion

Disperse the dried residue in 1ml of 12M sulphuric acid, using a vortex mixer. Leave at 35°C for 1 hour to solubilise the cellulose, then rapidly add 11ml of water and mix.

Heat the solution in a boiling water bath for 2 hours from re-boiling, stirring continuously. Cool it to room temperature by placing the tube in water, add 2ml of internal standard (2 mg of allose per ml of saturated benzoic acid solution) and mix the contents of the tube. Use 1ml of the hydrolysate for the preparation of alditol acetates and keep the remainder for the determination of uronic acids.

### Uronic acids

The method used is a modification of the method of Scott. Mix 0.3ml of hydrolysate (diluted, if necessary, so that it contains between 25 and 100µg of uronic acids per ml) with 0.3ml of a mixtures of sodium chloride-boric acid solution (prepared by adding 2g of sodium chloride and 3g of boric acid to 100ml of water) Add 5ml of concentrated sulphuric acid and vortex mix, then place the tube in a heating block at 70°C. Leave the tube and contents for 40 minutes and then cool them to room temperature by placing in water. When cool, add 0.2ml of 3.5-dimethylphenol solution (0.1g of (CH₃)₂-C₆H₃OH in 100ml of glacial acetic acid) and mix immediately. Between 10 and 15 minutes later read the absorbance at 400 and 450nm in a spectrophotometer against a water reference. Subtract the reading at 400nm from that at 450nm for each sample and plot the difference obtained for glucuronic acid standards (over the range 25-125µf ml-¹). Read the sample concentrations from the graph.

### Preparation of alditol acetates

To 1ml of hydrolysate add 0.2ml of 12M ammonia solution and 5µl of octan-2-ol. Test that the solution is alkaline, and then add 0.1ml of a freshly prepared solution of 100mg of sodium tetrahydroborate (III) (sodium borohydride) per ml of 3M ammonia solution. Mix, leave the mixture for 1 hour at 40°C and add 0.1ml of glacial acetic acid. Next, to 0.2ml of the acidified solution add 0.3ml of *N*-methylimidazole and 2ml of acetic anhydride, and mix. Leave it for 10 minutes at 20°C (room temperature), add 5ml of water, mix, and when cooled add 1ml of dichloromethane, agitate the contents vigorously on a vortex mixer and centrifuge for a few minutes to separate the mixture into two phases. Remove the bulk of the upper phase by aspiration and discard it, then transfer the lower phase to a small vial, seal and store it at -20°C. Use 1-2µl for injection on to the chromatograph.

### Alternative preparative of alditol acetates

When dichloromethane is used as a solvent for the alditol acetates it has been observed in a number of laboratories without automatic GLC injection facilities that the injection technique is critical to the obtaining of reproducible results. A more robust method can be obtained if dichloromethane is replaced with ethyl acetate as a solvent for alditol acetates. The procedure is as follows:
To 1ml of hydrolysate add 0.2ml of 12M ammonia solution and 5µl of octan-2-ol. Test that the solution is alkaline, then add 0.1ml of a freshly prepared solution of 100mg of sodium tetrahydroborate (III) per ml of 3M ammonia solution. Mix, leave the mixture for 1 hour at 40°C and add 0.1ml of glacial acetic acid. To 0.5ml of the acidified solution add 0.5ml of *N*-methylimidazole, 5ml of acetic anhydride and mix. Leave for 10 minutes at 20°C (room temperature), then add 0.6ml of ethanol and mix. After 5 minutes add 5ml of water, place in a water bath at room temperature, add 5ml of 7.5M KOH and a few minutes later a further 5ml of 7.5M KOH. Mix by inverting and leave to separate into two Phases. Transfer the top phase to a small vial and store at +5°C. Use 1-2µl for injection on the chromatogaph.

## Claims

1. Use of coconut endosperm fibre in the manufacture of a composition for the prevention or treatment of pathogenic bacteria in the large intestine of a dog or a cat.

2. Use, as claimed in claim 1 wherein the pathogenic bacteria is *Campylobacter,* pathogenic *Escherichia coli* or *Salmonella.*

3. Use, as claimed in claim 2, wherein the pathogenic bacteria is the species *Campylobacter jejuni*, or the species *Salmonella enterica.*

4. Use, as claimed in any one of the preceding claims, wherein the composition is a pet food product.

## Patentansprüche

1. Verwendung von Kokosnussendospermfaser bei der Herstellung einer Zusammensetzung zur Prävention oder Behandlung pathogener Bakterien im Dickdarm eines Hundes oder einer Katze.

2. Verwendung nach Anspruch 1, wobei die pathogenen Bakterien *Campylobacter*, pathogene *Escherichia coli* oder *Salmonella* sind.

3. Verwendung nach Anspruch 2, wobei die pathogenen Bakterien die Spezies *Campylobacterjejuni* oder die Spezies *Salmonella enterica* sind.

4. Verwendung nach einem der vorangehenden Ansprüche wobei die Zusammensetzung ein Haustierfutterprodukt ist.

## Revendications

1. Utilisation d'une fibre d'endosperme de noix de coco dans la fabrication d'une composition pour la prévention ou le traitement d'une bactérie pathogène dans le gros intestin d'un animal de compagnie.

2. Utilisation selon la revendication 1, dans laquelle la bactérie pathogène est *Campylobacter*, *Escherichia coli* pathogène ou *Salmonella.*

3. Utilisation selon la revendication 2, dans laquelle la bactérie pathogène est l'espèce *Campylobacter jejuni*, ou l'espèce *Salmonella enterica.*

4. Utilisation selon l'une quelconque des revendications précédentes, dans laquelle l'animal de compagnie est un chien ou un chat.
